# FASCICULE DE BREVET EUROPEEN

(11) **EP 0 625 973 B1**
(45) Date de publication et mention de la délivrance du brevet: **01.05.1996**
(21) Numéro de dépôt: 93902313.1
(22) Date de dépôt: 09.12.1992
(51) Int. Cl.: C07D 277/82, C07F 7/08

(54) **PROCEDE DE PREPARATION DE DERIVES D'AMINO-2 NITRO-4 BENZOTHIAZOLE ET INTERMEDIAIRES**
VERFAHREN ZUR HERSTELLUNG VON 2-AMINO-4-NITROBENZOTHIAZOL-DERIVATEN UND ZWISCHENPRODUKTE
METHOD FOR PREPARING 2-AMINO-4-NITROBENZOTHIAZOLE DERIVATIVES AND INTERMEDIATES

(30) Priorité: 13.12.1991 FR 9115487
(43) Date de publication de la demande: 30.11.1994
(73) Titulaire: RHONE-POULENC RORER S.A., F-92160 Antony (FR)
(72) Inventeur: AUDIAU, François, F-94220 Charenton-le-Pont (FR); JIMONET, Patrick, F-78450 Villepreux (FR); MIGNANI, Serge, F-94290 Ch tenay-Malabry (FR)
(74) Mandataire: Savina, Jacques
(86) Numéro de dépôt international: FR9201166
(87) Numéro de publication internationale: WO9312100

(56) Documents cités:
- EP-A- 0 374 041
- CHEMICAL ABSTRACTS, vol. 118, no. 1, 4 Janvier 1993, Columbus, Ohio, US; abstract no. 6906b, S. MIGNANI et al.: "Versatile methods for the synthesis of 2-amino-6-(trifluoromethoxy)nitrobenzothiazoles", page 735 ;

## Description

La présente invention concerne un procédé de préparation de dérivés d'amino-2 nitro-4 benzothiazole de formule : 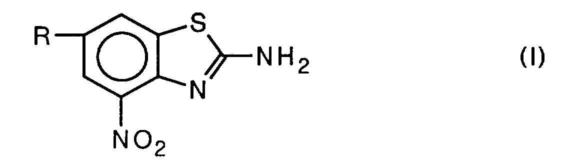 dans laquelle R représente un radical alkyle, alcoxy, alkylthio, polyfluoroalkyle, polyfluoroalcoxy, alcényle, phényle, alkylsulfonyle, alcoxycarbonyle, amino, cyano, sulfonamido ou dialkylcarbamoyle.

Il est connu du brevet EP 374041 de préparer certains composés de formule (I) par nitration, au moyen d'acide nitrique, des amino-2 benzothiazoles correspondants; cependant ces composés sont obtenus en mélange avec les dérivés nitrés en position -5 et avec de très faibles rendements. Ce procédé n'est donc pas applicable industriellement.

Il a maintenant été trouvé et c'est ce qui fait l'objet de la présente demande, un nouveau procédé permettant d'obtenir industriellement et avec de bons rendements les composés de formule (I).

Ce procédé consiste à faire réagir un dérivé de formule : 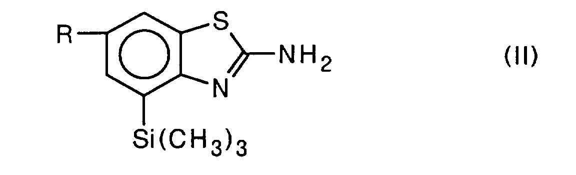 dans laquelle R a les mêmes significations que dans la formule (I) avec le tétrafluoroborate de nitronium.

Cette réaction s'effectue au sein d'un solvant organique inerte tel que l'acétonitrile, le tétraméthylènesulfone.

Il est particulièrement avantageux d'opérer à une température comprise entre 10°C et 30°C et, de préférence, à environ 20°C.

La quantité de tétrafluoroborate de nitronium est généralement de 1 mole à 4 moles pour une mole de dérivé de formule (II). De façon avantageuse, on en utilise 2 moles.

La durée de la réaction est comprise entre 6 et 48 heures. Elle est généralement de 12 heures.

Les dérivés de formule (II) sont nouveaux et font également partie de l'invention.

Les dérivés de formule (II) peuvent être obtenus par action de triméthylchlorosilane sur les dérivés lithiés des amino-2 benzothiazoles correspondants, ces derniers étant obtenus par action de n-butyllithium sur les amino-2 bromo-4 benzothiazoles.

Cette réaction s'effectue sans séparation du dérivé lithié, au sein d'un solvant inerte tel que l'hexane, le tétrahydrofuranne ou un mélange de ces solvants, à une température voisine de -80°C.

Les amino-2 bromo-4 benzothiazoles peuvent être obtenus par bromation des amino-2 benzothiazoles correspondants.

Cette bromation s'effectue généralement au moyen de brome, au sein de l'acide acétique, à une température comprise entre 20°C et 60°C.

Les amino-2 benzothiazoles peuvent être obtenus selon la méthode décrite par L. M. YAGUPOLSKII et coll., Zh. Obshch. Khim., 33 (7), 2301 (1963).

Les composés de formule (I) peuvent être séparés du mélange réactionnel selon les techniques habituelles de séparation (extraction, chromatographie, cristallisation par exemple).

Les dérivés de formule (I) sont utiles en tant que médicaments ou intermédiaires pour la préparation de médicaments (EP282971 et EP 374041).

### EXEMPLE 1

A une solution de 0,76 g d'amino-2 triméthylsilyl-4 trifluorométhoxy-6 benzothiazole dans 20 cm3 d'acétonitrile refroidie à 0°C on ajoute 0,68 g de tétrafluoroborate de nitronium, en petites portions. L'agitation est poursuivie pendant 12 heures à une température voisine de 20°C. La solution est ensuite diluée avec 50 cm3 d'eau et extraite avec 2 fois 50 cm3 d'acétate d'éthyle. Les phases organiques sont réunies, séchées sur sulfate de sodium et concentrées sous vide. Le résidu est purifié par flash chromatographie sur gel de silice avec un mélange cyclohexane-acétate d'éthyle (7-3 en volumes) comme éluant. Après recristallisation dans un mélange cyclohexane-acétate d'éthyle (3-2 en volumes), on obtient 0,3 g d'amino-2 nitro-4 trifluorométhoxy-6 benzothiazole fondant à 260°C.

L'amino-2 triméthylsilyl-4 trifluorométhoxy-6 benzothiazole peut être préparé de la manière suivante : 18,7 cm3 de n-butyllithium (1,6 M dans l'hexane) sont ajoutés goutte à goutte, en 1 heure à une solution de 3,13 g d'amino-2 bromo-4 trifluorométhoxy-6 benzothiazole dans 50 cm3 de tétrahydrofuranne sous azote. La solution est agitée 45 minutes à -78°C puis 6,5 g de triméthylchlorosilane sont ajoutés goutte à goutte pendant 15 minutes. Le mélange est agité 1 heure à 0°C, dilué avec 100 cm3 d'eau et extrait avec 2 fois 100 cm3 d'acétate d'éthyle. Les phases organiques sont réunies, séchées sur sulfate de sodium et évaporées sous pression réduite. Le résidu est purifié par flash chromatographie sur gel de silice avec un mélange cyclohexane-acétate d'éthyle (92-8 en volumes) comme éluant. Après recristallisation dans un mélange dichlorométhane-méthanol (95-5 en volumes), on obtient ainsi 1,6 g d'amino-2 triméthylsilyl-4 trifluorométhyl-6 benzothiazole fondant à 76°C.

L'amino-2 bromo-4 trifluorométhoxy-6 benzothiazole peut être obtenu de la manière suivante : 2,4 g de brome dans 6 cm3 d'acide acétique sont ajoutés, goutte à goutte, en 15 minutes, à une solution agitée à 50°C de 3,51 g d'amino-2 trifluorométhoxy-6 benzothiazole dans 30 cm3 d'acide acétique. Le milieu réactionnel est agité à 50°C pendant 12 heures. Après filtration et recristallisation dans l'éthanol, on obtient 4,5 g de bromhydrate d'amino-2 bromo-4 trifluorométhoxy-6 benzothiazole fondant à 300°C.

L'amino-2 trifluorométhoxy-6 benzothiazole peut être préparé selon la méthode décrite par L. M. YAGUPOLSKII et coll., Zh. Obshch. Khim., 33 (7), 2301 (1963).

## Revendications

1. Procédé de préparation de dérivés de formule : 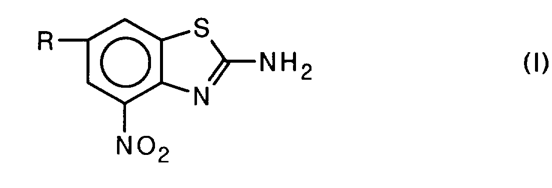 dans laquelle R représente un radical alkyle, alcoxy, alkylthio, polyfluoroalkyle, polyfluoroalcoxy, alcényle, phényle, alkylsulfonyle, alcoxycarbonyle, amino, cyano, sulfonamido ou dialkylcarbamoyle, caractérisé en ce que l'on fait réagir, au sein d'un solvant inerte, un dérivé de formule : 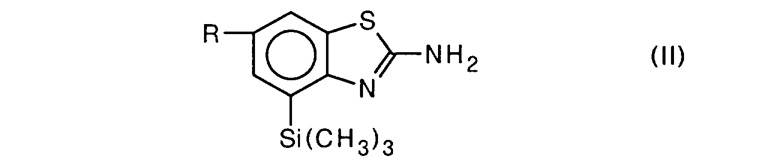 dans laquelle R a les mêmes significations que dans la formule (I) avec le tétrafluoroborate de nitronium.

2. Procédé selon la revendication 1 caractérisé en ce que le solvant inerte est l'acétonitrile ou le tétraméthylènesulfone.

3. Procédé selon l'une des revendications 1 ou 2 caractérisé en ce que l'on opère à une température comprise entre 10 et 30°C.

4. Procédé selon la revendication 3 caractérisé en ce que l'on opère à une température d'environ 20°C.

5. Procédé selon l'une des revendications 1 à 4 caractérisé en ce que l'on utilise 1 à 4 moles de tétrafluoroborate de nitronium par mole de dérivé de formule (II).

6. Procédé selon la revendication 5 caractérisé en ce que l'on utilise 2 moles de tétrafluoroborate de nitronium.

7. Procédé selon l'une des revendications 1 à 6 caractérisé en ce que la durée de la réaction est comprise entre 6 et 48 heures.

8. Composés de formule : 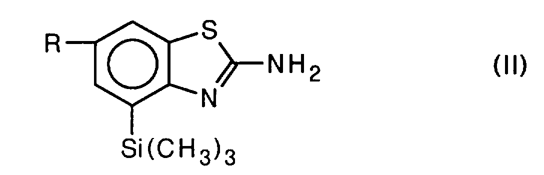 dans laquelle R représente un radical alkyle, alcoxy, alkylthio, polyfluoroalkyle, polyfluoroalcoxy, alcényle, phényle, alkylsulfonyle, alcoxycarbonyle, amino, cyano, sulfonamido ou dialkylcarbamoyle.

## Patentansprüche

1. Verfahren zur Herstellung von Derivaten der Formel 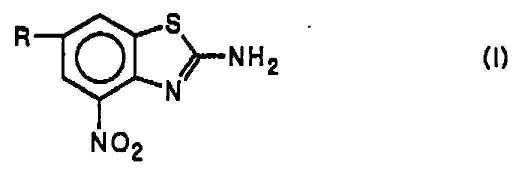 worin R einen Alkyl-, Alkoxy-, Alkylthio-, Polyfluoroalkyl-, Polyfluoroalkoxy-, Alkenyl-, Phenyl-, Alkylsulfonyl-, Alkoxycarbonyl-, Amino-, Cyano-, Sulfonamido- oder Dialkylcarbamoylreat darstellt, dadurch gekennzeichnet, daß man in einem inerten Lösungsmittel ein Derivat der Formel 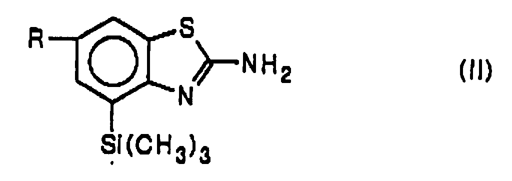 worin R die gleichen Bedeutungen wie in Formel (I) hat, mit Nitroniumtetrafluoroborat zur Reaktion bringt.

2. Verfahren gemäß Anspruch 1, dadurch gekennzeichnet, daß das inerte Lösungsmittel Acetonitril oder Tetramethylensulfon ist.

3. Verfahren gemäß einem der Ansprüche 1 oder 2, dadurch gekennzeichnet, daß man bei einer Temperatur zwischen 10 °C und 30 °C arbeitet.

4. Verfahren gemäß Anspruch 3, dadurch gekennzeichnet, daß man bei einer Temperatur von etwa 20 °C arbeitet.

5. Verfahren gemäß einem der Ansprüche 1 bis 4, dadurch gekennzeichnet, daß man 1 Mol bis 4 Mol Nitroniumtetrafluoroborat pro Mol des Derivats der Formel (II) verwendet.

6. Verfahren gemäß Anspruch 5, dadurch gekennzeichnet, daß man 2 Hol Nitroniumtetrafluoroborat verwendet.

7. Verfahren gemäß einem der Ansprüche 1 bis 6, dadurch gekennzeichnet, daß die Dauer der Reaktion zwischen 6 Stunden und 48 Stunden beträgt.

8. Verbindungen der Formel (II) 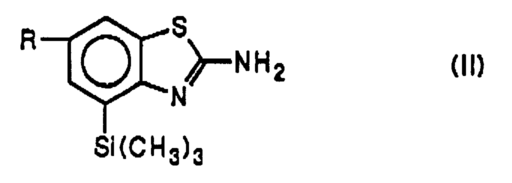 worin R einen Alkyl-, Alkoxy-, Alkylthio-, Polyfluoroalkyl-, Polyfluoroalkoxy-, Alkenyl-, Phenyl-, Alkylsulfonyl-, Alkoxycarbonyl-, Amino-, Cyano-, Sulfonamido- oder Dialkylcarbamoylrest darstellt.

## Claims

1. Process for the preparation of derivatives of formula: 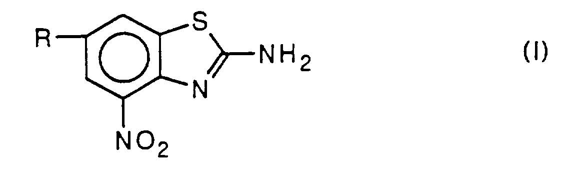 in which R denotes an alkyl, alkoxy, alkylthio, polyfluoroalkyl, polyfluoroalkoxy, alkenyl, phenyl, alkylsulphonyl, alkoxycarbonyl, amino, cyano, sulphonamido or dialkylcarbamoyl radical, characterized in that a derivative of formula: 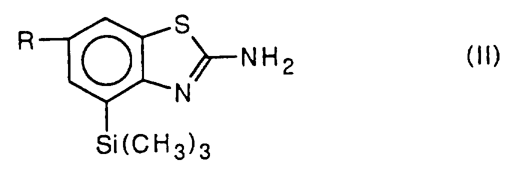 in which R has the same meanings as in formula (I) is reacted in an inert solvent, with nitronium tetrafluoroborate.

2. Process according to Claim 1, characterized in that the inert solvent is acetonitrile or tetramethylene sulphone.

3. Process according to one of Claims 1 or 2, characterized in that the operation is carried out at a temperature of between 10 and 30°C.

4. Process according to Claim 3, characterized in that the operation is carried out at a temperature of approximately 20°C.

5. Process according to one of Claims 1 to 4, characterized in that 1 to 4 moles of nitronium tetrafluoroborate are employed per mole of derivative of formula (II).

6. Process according to Claim 5, characterized in that 2 moles of nitronium tetrafluoroborate are employed.

7. Process according to one of Claims 1 to 6, characterized in that the reaction period is between 6 and 48 hours.

8. Compounds of formula: 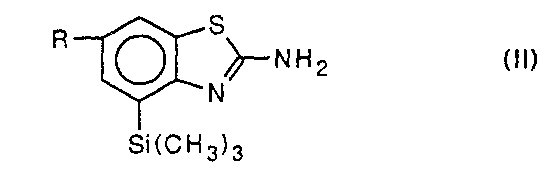 in which R denotes an alkyl, alkoxy, alkylthio, polyfluoroalkyl, polyfluoroalkoxy, alkenyl, phenyl, alkylsulphonyl, alkoxycarbonyl, amino, cyano, sulphonamido or dialkylcarbamoyl radical.
